# EUROPÄISCHE PATENTSCHRIFT

(11) **EP 0 597 071 B1**
(45) Veröffentlichungstag und Bekanntmachung des Hinweises auf die Patenterteilung: **18.04.2001**
(21) Anmeldenummer: 93912552.2
(22) Anmeldetag: 28.05.1993
(51) Int. Cl.: A61K 31/565

(54) **ANDROGENE ZUR STIMULIERUNG DES ZENTRALNERVENSYSTEMS**
ANDROGENS FOR STIMULATING THE CENTRAL NERVOUS SYSTEM
ANDROGENES VISANT A STIMULER LE SYSTEME NERVEUX CENTRAL

(30) Priorität: 03.06.1992 DE 4218292
(43) Veröffentlichungstag der Anmeldung: 18.05.1994
(73) Patentinhaber: CUM Pharma Consulting Anstalt, 9490 Vaduz (CH)
(72) Erfinder: MATTERN, Claudia, D-8130 Starnberg (DE); HÄCKER, Rüdiger, D-82211 Herrsching (DE)
(74) Vertreter: Winkler, Andreas, Dr.
(86) Internationale Anmeldenummer: DE9300473
(87) Internationale Veröffentlichungsnummer: WO9324128

(56) Entgegenhaltungen:
- DE-A- 2 345 376
- ZSCHR. AERZTL. FORTBILD. Bd. 67, Nr. 6, 1973, Seiten 277 - 279 G. M]LLER 'Erfahrungen }ber die Therapie mit anabolen Hormonen bei Hormonen bei der Osteoporose'
- ZEITSCHRIFT F}R ALTERSFORSCHUNG Bd. 33, Nr. 2, 1978, Seiten 167 - 172 U.J. SCHMIDT ET AL. 'Bone diseases in advanced and old age: diagnostics, prophylaxis, therapy'
- DEUTSCHES GESUNDHEITSW. Bd. 22, Nr. 27, 1967, Seiten 1276 - 1279 J. MACH 'Praktische Erfahrungen mit Turinabol bei der Osteoporosebehandlung'
- Auszug aus dem Arzneimittelverzeichnis der DDR, Eintrag "Oral-Turinabol"

## Beschreibung

Die Erfindung betrifft ein Arzneimittel, das neben seiner bereits bekannten Wirkung im Stoffwechsel in der Lage ist, den Aktivierungszustand des Zentralnervensystems positiv zu beeinflussen bzw. physiologisch zu optimieren.

Die psychophysiologische und psychische Leistungsfähigkeit des Menschen wird neben anderen Faktoren wesentlich durch den Aktivierungszustand des Zentralnervensystems bestimmt. Diese Aktivierung läßt sich anhand der Verschiebung der mittleren alpha-Frequenz im Elektroenzephalogramm beurteilen. Jede physische oder psychische Beanspruchung des Menschen führt zu einer Erhöhung des Frequenz um etwa 4 bis 6 Hertz über dem individuellen Ausgangswert. Diese Zunahme geht einher mit einer Verbesserung der psychophysiologischen Leistungsfähigkeit. Wenn die Beanspruchung zu stark ist oder sie nicht adaequat bewältigt werden kann, vermindert sich die alpha-Frequenz nach Durchlaufen eines Optimums. Dadurch nimmt die psychophysiologische Leistungsfähigkeit ab und kann unter den Ausgangswert vor Beginn der Beanspruchung sinken.

Es konnte bei Untersuchungen an Spitzensportlern nachgewiesen werden, daß die Zunahme der alpha-Frequenz schon durch die geistige Konzentration auf den Vollzug der Leistung ausgelöst wird. Gleiche Wirkung hat eine angemessene körperliche Tätigkeit vor Bewältigung der eigentlichen Beanspruchung. Wird das Optimum der Aktivierung durch falsche Vorbereitung, Überbeanspruchung, Angstsituationen oder störende äußere Einflüsse überschritten, sinkt die Leistungsfähigkeit, Fehler häufen sich, Korrekturen sind mit mehr psychischem und physischem Aufwand verbunden. Der Grad der Erschöpfung nach Vollzug der Leistung ist höher.

Im mittleren und höheren Lebensalter ist eine Ursache der nachlassenden Leistungsfähigkeit eine nicht belastungsadaequate Aktivierung des Zentralnervensystems. In Phasen längerfristiger und hoher psychophysiologischer Beanspruchung und besonders bei eingeschränkter Aktivierung kommt damit der Einstellung und Aufrechterhaltung eines optimalen Aktivierungszustandes des Zentralnervensystems in diesem Lebensalter eine besondere Bedeutung für Erhalt und Optimierung der Leistungsfähigkeit zu.

Aus der DE-A-23 45 376 ist die Verwendung von Mesterolon, d.h. 17ß-Hydroxy-1α-methyl-5α-androstan-3-on und seiner Ester in Arzneimitteln mit neuropsychotroper Wirkung bekannt.

Überraschenderweise hat es sich nunmehr gezeigt, daß ein optimaler Aktivierungszustand des Zentralnervensystems durch die Applikation eines Arzneimittels mit einem Gehalt an Mestanolon und/oder 4-Chlor-1-dehydromethyltestosteron (Oral-Turinabol®) erreicht werden kann.

Dabei kann das Arzneimittel als Depotform, bevorzugt Retarddragee, oder Buccaltablette oder in Form einer galenischen Zubereitungsform, die die pernasale Applikation mittels Nasenspray erlaubt, eingesetzt werden.

Bevorzugt ist ein Gehalt an Mestanolon und/oder 4-Chlor-1-dehydromethyltestosteron (Oral-Turinabol®) von 5 bis 20 mg pro Einnahmeeinheit.

Mestanolon (STS 646, 17β-Hydroxy-17-methyl-5α-androstan-3-on) ist ein Derivat des Testosterons. Durch die chemische Modifikation wird die vorherrschende androgene Wirksamkeit des Wirkstoffes zurückgedrängt und die anabole Wirksamkeit betont. Der Mestanolon ist in Griechenland unter der Indikationsstellung "Anabolikum/Androgen" zugelassen.

Überraschenderweise hat sich ergeben, daß - verglichen mit anderen anabol wirksamen Steroiden (z.B. Dianabol), die therapeutisch eingesetzt werden - die anabole Wirksamkeit von Mestanolon deutlich geringer ist. Bei Applikation einer gleichen Dosis (bspw. 10-20 mg/pro Tag) tritt unter Mestanolon eine Verminderung der Harnstoffkonzentration im Blut ein; sie ist jedoch mit 4 % deutlich geringer als bei den anderen Steroiden (bis zu 30 %).

Untersuchungen an physisch hochbelasteten Probanden zeigten, daß bei Zufuhr von Mestanolon der Proteinstoffwechsel im Gleichgewicht bleibt und weder während noch nach der Belastung in katabole Situationen gerät. Eine deutliche Betonung der anabolen Situation wurde jedoch nicht nachgewiesen.

Bei diesen Untersuchungen wurde völlig unerwartet und überraschend eine bislang unbekannte weitere Wirkung des Mestanolons entdeckt - die Zufuhr führte zu einer Optimierung der zentralnervalen Aktivierung während der Vorbereitung auf die Leistung und zu einem Erhalt dieser optimalen Aktivierung auch bei hohen Belastungen. Daraus resultierte eine erhöhte psychophysiologische Leistungsfähigkeit.

54 über einen längeren Zeitraum hochbelastete Probanden wiesen nach Applikation von 10 mg Mestanolon pro Tag eine deutliche Zunahme der alpha-Frequenz um bis zu 4 Hertz auf. Damit verbunden war eine Regularisierung und Optimierung des Frequenzanstiegs unter Belastung. Die untersuchten Probanden absolvierten schwierige Übungen, die hohe körperliche Leistungen verbunden mit hohen Ansprüchen an geistige Konzentration und muskuläre Koordination erforderten, mit deutlich weniger Fehlern. Komplizierte Bewegungsabläufe mit einem hohen Grad an Beanspruchung führten nicht zu einer Verminderung des Aktivierungsgrades, sondern die erhöhte Leistungsfähigkeit blieb auch nach mehrfachen Wiederholungen erhalten.

Die Zufuhr von Mestanolon ist daher geeignet, in Phasen hoher Belastung und insbesondere beim Menschen im mittleren und höheren Lebensalter mit Defiziten in der Aktivierung, eine belastungsadaequate Optimierung des zentralnervalen Aktivierungszustands auszulösen und zu stützen.

Aufgrund dieser Ergebnisse wurden vergleichende Untersuchungen an 10 Probanden durchgeführt, die sich 4-6 Stunden am Tag hohen und erschöpfenden körperlichen Belastungen unterzogen. 5 Probanden nahmen über sechs Wochen täglich zwischen 5 - 10 mg 4-Chlor-1-dehydromethyltestosteron (Oral-Turinabol®) zu sich, um eine ausreichende Wiederherstellung und Belastbarkeit zu sichern. Die anderen 5 Probanden erhielten ein Plazebo. Die anabole Wirkung der Medikation war anhand der Konzentration der Muskeleiweiße und des Harnstoffes im Serum wie erwartet nachweisbar. Überraschenderweise wurde jedoch, verglichen mit den Probanden, die Plazebo erhielten, auch eine deutliche Zunahme der zentralnervalen Aktivierung bei der Mehrzahl der mit 4-Chlor-1-dehydromethyltestosteron (Oral-Turinabol®) behandelten Probanden gefunden. Diese Seitenwirkung des 4-Chlor-1-dehydromethyltestosteron (Oral-Turinabols®) war bislang nicht bekannt.

Die Applikation des Wirkstoffes ist sowohl in Form von Depotformen wie Retarddragees oder Buccaltabletten möglich. Besonders vorteilhaft ist es aber, wenn das Arzneimittel in Form eines Nasensprays pernasal appliziert wird. Es liegen Hinweise vor, daß diese pernasale Applikation den Durchtritt durch die Blut-Hirnschranke erleichtert. Damit gelangt der Wirkstoff schneller und unter Umgehung des "first-pass-Metabolismus" in der Leber an das Erfolgsorgan, die im Zentralnervensystem vorhandenen Steroidrezeptoren. Verbunden mit dieser Umgehung ist eine Reduktion der erforderlichen Dosis pro Einzelapplikation auf ca. 5 mg. Durch Reduktion der Dosis und Umgehung der Leber werden negative Einflüsse auf den Leberstoffwechsel, die laut Literatur durch die 17-alpha-Methylierung verursacht werden könnten, vermieden.

Eine weitere besonders vorteilhafte Eigenschaft der erfindungsgemäßen weiteren medizinischen Indikation von Mestanolon und 4-Chlor-1-dehydromethyltestosteron (Oral-Turinabol®) ist darin zu sehen, daß diese Substanzen im Gegensatz zu anderen anabol wirksamen Steroiden auch bei Applikation über einen längeren Zeitraum (20 mg pro Tag über 6 Wochen) keine negativen Rückwirkungen auf die endogene Regulation des Testosteronspiegels zeigt. Auch dies war vor dem Hintergrund des bekannten Verhaltens anderer Anabolika eine überraschende Beobachtung.

Die Zufuhr von von Mestanolon und/oder 4-Chlor-1-dehydromethyltestosteron (oral-Turinabol®) bewirkt eine Optimierung der zentralnervalen Aktivierung ohne negative Beeinflussung der endogenen Regulation des Testosteronspiegels und damit des individuellen anabol/katabolen Gleichgewichtes im Stoffwechsel. Von besonderer Bedeutung ist es, daß eine Langzeitanwendung ohne schädliche Rückwirkungen möglich ist. Der oben beschriebene positive Effekt auf das Zentralnervensystem wirkt sich dabei zusätzlich vorteilhaft aus.

## Patentansprüche

1. Verwendung von Mestanolon und/oder 4-Chlor-1-dehydrome-thyltestosteron zur Herstellung eines Arzneimittels zur Erhöhung der psychophysiologischen Leistungsfähigkeit durch Optimierung des Aktivierungsgrades des zentralen Nervensystems.

2. Verwendung nach Anspruch 1, dadurch gekennzeichnet, daß das Arzneimittel als Depotform oder Buccaltablette eingesetzt wird.

3. Verwendung nach Anspruch 2, dadurch gekennzeichnet, daß das Arzneimittel als Retarddragee eingesetzt wird.

4. Verwendung nach Anspruch 1, gekennzeichnet durch eine galenische Zubereitungsform des Arzneimittels, die die pernasale Applikation mittels Nasenspray erlaubt.

5. Verwendung nach einem der vorangehenden Ansprüche, gekennzeichnet durch einen Gehalt an Mestanolon und/oder 4-Chlor-1-dehydromethyltestosteron im Arzneimittel von 5 bis 20 mg pro Einnahmeeinheit.

## Claims

1. Use of mestanolone and/or 4-chloro-1-dehydromethyl testosterone for producing a drug for increasing the psychophysiological capacity by optimising the degree of activation of the central nervous system.

2. Use according to claim 1, characterised in that the drug is used in depot form or in the form of buccal tablets.

3. Use according to claim 2, characterised in that the drug is used in the form of a retard dragee.

4. Use according to claim 1, characterised by a galenic preparation of the drug in the form of a nose spray for pernasal application.

5. Use according to any of the preceding claims, characterised in that the content of mestanolone and/or 4-chloro-1-dehydromethyl testosterone in the drug is 5 to 20 mg per ingestion unit.

## Revendications

1. Utilisation de Mestanolon et/ou de 4-chloro-1-dehydrométhyltestostérone pour la fabrication d'un médicament destiné à augmenter la capacité psychophysiologique en optimisant le degré d'activation du système nerveux central.

2. Utilisation selon la revendication 1, caractérisée en ce que le médicament est utilisé comme forme à effet de longue durée ou comme comprimé.

3. Utilisation selon la revendication 2, caractérisée en ce que le médicament est utilisé comme dragée à effet différé.

4. Utilisation selon la revendication 1, caractérisée par une forme de préparation galénique du médicament qui permet l'application pernasale au moyen d'un spray nasal.

5. Utilisation selon l'une des revendications précédentes, caractérisée par une teneur en Mestanolon et/ou en 4-chloro-1-dehydrométhyltestostérone dans le médicament de 5 à 20 mg par dose unitaire.
